# EUROPEAN PATENT APPLICATION

(11) **EP 4 140 506 A1**
(43) Date of publication of application: **01.03.2023**
(21) Application number: 21816858.1
(22) Date of filing: 01.06.2021
(51) Int. Cl.: A61L 2/00, A61M 1/36, A61M 1/14

(54) **STERILIZATION MODULE AND STERILIZATION SYSTEM COMPRISING SAME**

(30) Priority: 01.06.2020 US 202063032799 P; 28.05.2021 US 202163194512 P
(71) Applicant: Seoul Viosys Co., Ltd, Ansan-si, Gyeonggi-do 15429 (KR)
(72) Inventor: YOON, Yeong Min, Ansan-si Gyeonggi-do 15429 (KR); BAE, Hee Ho, Ansan-si Gyeonggi-do 15429 (KR)
(74) Representative: Stolmár & Partner Patentanwälte PartG mbB
(86) International application number: PCT/KR2021/006819
(87) International publication number: WO 2021/246761

(57) **Abstract**

Disclosed herein are a sterilization module and a sterilization system including the same. The sterilization module includes a light source emitting germicidal light to kill pathogens in living cells. The living cells may include living cells and the pathogens. In addition, the germicidal light may be light in a wavelength band capable of ensuring a viability of 70% or more for the normal cells and a viability of less than 70% for the pathogens.

## Description

### [Technical Field]

Embodiments of the present disclosure relate to a sterilization module and a sterilization system including the same.

### [Background Art]

Different wavelengths of UV light have different properties. Particularly, UV light with a wavelength having germicidal properties is used in a sterilization module.

Recently, sterilization modules using various types of UV light have been developed and used to sterilize a variety of objects. Such a sterilization module may be housed in a specific device such as a refrigerator, a washing machine, a humidifier, and a water purifier and may also be attached to the human body such as a nasal cavity or an ear canal.

In addition, UV light is used for the purpose of killing pathogens to treat inflammations and the like.

In general, UV light used to kill microorganisms such as bacteria and viruses is short-wavelength UV light such as UVB or UVC. UVB or UVC kills microorganisms by destroying DNA thereof.

However, such UVB or UVC-based sterilization can have harmful effects on normal cells in the human body, as well as on the microorganisms.

That is, when UVB or UVC is delivered to the human body for therapeutic purposes, there is concern of destroying DNA in normal cells of body tissue apart from pathogens.

The above information disclosed in this section is only to aid in understanding of the background of the inventive concepts and thus may contain information that does not constitute prior art.

### [Disclosure]

### [Technical Problem]

Embodiments of the present disclosure provide a sterilization module that can kill pathogens and a sterilization system including the same.

Embodiments of the present disclosure provide a sterilization module that can kill only pathogens while minimizing damage to normal living cells and a sterilization system including the same.

### [Technical Solution]

In accordance with one aspect of the present disclosure, a sterilization module includes a light source emitting germicidal light to kill pathogens in living cells. Here, the living cells may include normal cells and the pathogens. The germicidal light may be light in a wavelength band capable of ensuring a viability of 70% or more for the normal cells and a viability of less than 70% for the pathogens.

The germicidal light may be light in a wavelength band capable of ensuring a viability of 80% or more for the normal cells and a viability of 20% or less for the pathogens.

The germicidal light may ensure a tail intensity of less than 8% for the living cells. Here, the tail intensity may be a percentage of DNA in a comet tail resulting from DNA strand breaks in the living cells.

The germicidal light may be light having a peak at a wavelength of 405 nm.

The light source may deliver the germicidal light to the living cells at an energy density of greater than 6 J/cm² to less than 20 J/cm².

The light source delivers the germicidal light to the living cells at an energy density of 10 J/cm².

The sterilization module may deliver the germicidal light to body tissue or blood discharged from the human body.

In accordance with another aspect of the present disclosure, a sterilization system includes a sterilization module emitting germicidal light to kill pathogens in living cells. Here, the living cells may include normal cells and the pathogens. The germicidal light may be light in a wavelength band capable of ensuring a viability of 70% or more for the normal cells and a viability of less than 70% for the pathogens.

The germicidal light may be light in a wavelength band capable of ensuring a viability of 80% or more for the normal cells and a viability of 20% or less for the pathogens.

The germicidal light may ensure a tail intensity of less than 8% for the living cells. Here, the tail intensity may be a percentage of DNA in a comet tail resulting from DNA strand breaks in the living cells.

The germicidal light may be light having a peak at a wavelength of 405 nm.

The sterilization module may deliver the germicidal light to the living cells at an energy density of greater than 6 J/cm² to less than 20 J/cm².

The sterilization module may deliver the germicidal light to the living cells at an energy density of 10 J/cm².

The sterilization module of the sterilization system may deliver the germicidal light to body tissue or blood discharged from the human body.

For example, the sterilization system may further include a hemodialysis device for dialysis of the blood discharged from the human body.

### [Advantageous Effects]

The sterilization module and the sterilization system according to the embodiments can kill pathogens in living tissue or blood using germicidal light.

In addition, the sterilization module and the sterilization system according to the embodiments can provide healthy blood to a patient by killing only pathogens while minimizing damage to normal living cells.

Further, the sterilization module and the sterilization system according to the embodiments can minimize side effects that occur when blood is injected into the human body by killing only pathogens in the blood while minimizing damage to normal living cells in the blood.

### [Description of Drawings]

FIG. 1 is a block diagram of a sterilization system according to a first embodiment of the present disclosure.
FIG. 2 is a schematic view of the sterilization module according to the first embodiment.
FIG. 3 is a graph showing the results of Experiment 1 to determine viability of normal cells and pathogens depending on light treatment conditions.
FIG. 4 is a graph showing the degree of DNA damage to normal cells in blood of a healthy subject depending on light treatment conditions.
FIG. 5 is a graph showing the degree of DNA damage to normal cells in blood of a diseased subject depending on light treatment conditions.
FIG. 6 is a graph showing the degree of DNA damage to normal cells in blood of a healthy subject depending on the energy density of a first type of light.
FIG. 7 is a graph showing the degree of DNA damage to normal cells in blood of a diseased subject depending on the energy density of the first type of light.
FIG. 8 is a block diagram of a sterilization system according to a second embodiment of the present disclosure.

### [Best Mode]

Reference will now be made in detail to various embodiments, examples of which are illustrated in the accompanying drawings. It should be understood that the embodiments are provided for complete disclosure and thorough understanding of the present disclosure by those skilled in the art. Therefore, the present disclosure is not limited to the following embodiments and may be embodied in different ways. In addition, the drawings may be exaggerated in width, length, and thickness of components for descriptive convenience and clarity only. Like components will be denoted by like reference numerals throughout the specification.

Hereinafter, a sterilization module and a sterilization system according to embodiments of the present disclosure will be described in conjunction with the accompanying drawings.

In the following detailed description of a sterilization module and a sterilization system according to the embodiments, the sterilization module is described as being applied to a hemodialysis system. However, it will be understood that this is for illustration only and the sterilization module according to the present disclosure is not necessarily applied only to the hemodialysis system.

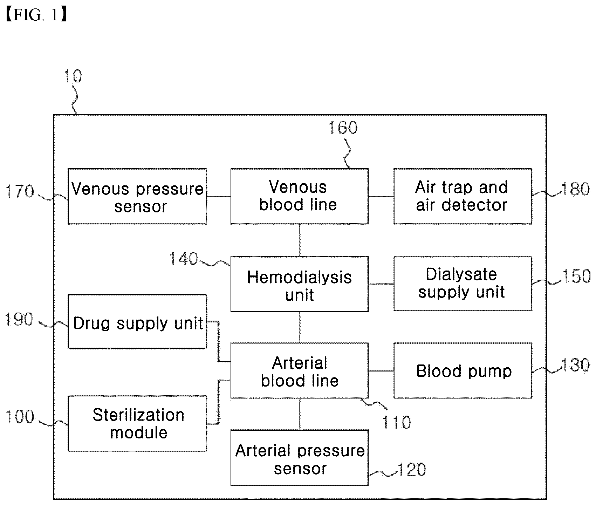

FIG. 1 is a block diagram of a sterilization system according to a first embodiment of the present disclosure.

That is, FIG. 1 is a block diagram of a sterilization system which is a hemodialysis system employing a sterilization module according to embodiments of the present disclosure.

Referring to FIG. 1, the sterilization system 10 according to the first embodiment includes an arterial blood line 110, an arterial pressure sensor 120, a blood pump 130, a hemodialysis unit 140, a dialysate supply unit 150, a venous blood line 160, a venous pressure sensor 170, an air trap and air detector 180, a drug supply unit 190, and a sterilization module 100.

The arterial blood line 110 is a passage along which blood from the artery of a patient moves to the hemodialysis unit 140. The arterial blood line 110 may include a catheter that is partially inserted into the artery of a patient.

The arterial pressure sensor 120 and the blood pump 130 may be connected to the arterial blood line 110.

The arterial pressure sensor 120 may detect the pressure and changes in pressure of the blood moving along the arterial blood line 110.

The blood pump 130 forces the blood extracted from a patient to be injected back into the vein of the patient through the arterial blood line 110, the hemodialysis unit 140, and the venous blood line 160.

For example, the blood pump 130 may force the blood extracted from a patient into the hemodialysis unit 140 through the arterial blood line 110. In addition, the blood pump 130 may force the blood purified by the hemodialysis unit 140 to be injected back into the patient.

The hemodialysis unit 140 removes waste matter from the blood. For example, the blood from the arterial blood line 110 is allowed to pass through the hemodialysis unit 140. Here, the hemodialysis unit 140 removes waste matter from the blood by inducing transport of the waste matter from the blood into dialysate through a semi-permeable membrane.

The dialysate supply unit 150 may store dialysate and may supply the dialysate to the hemodialysis unit 140.

The venous blood line 160 is a passage along which the blood with the waste matter removed therefrom by the hemodialysis unit moves to the vein of a patient. The venous blood line 160 may include a catheter that is partially inserted into the vein of a patient.

The venous pressure sensor 170 and the air trap and air detector 180 may be connected to the venous blood line 160.

The venous pressure sensor 170 may detect the pressure and changes in pressure of the blood moving along the venous blood line 160.

The air trap and air detector 180 may detect air in the blood moving along the venous blood line 160 and may remove the air.

The drug supply unit 190 may be connected to the arterial blood line 110.

The drug supply unit 190 may store a drug needed for hemodialysis. Thus, the drug supply unit 190 may supply the drug stored therein to the arterial blood line 110.

In addition, the drug supply unit 190 may receive a drug from outside of the sterilization system 10. Thus, the drug supply unit 190 may supply the drug received from the outside to the arterial blood line 110. For example, the drug supply unit 190 may supply heparin to the arterial blood line 110.

However, the drug that the drug supply unit 190 supplies is not necessarily limited to heparin. In addition, the drug supply unit 190 may be connected to other components apart from the arterial blood line 110 depending on the type of drugs used.

The sterilization module 100 may sterilize pathogens such as bacteria and viruses in the blood. That is, the sterilization module 100 may sterilize pathogens in the blood from a patient to supply safe blood to the patient.

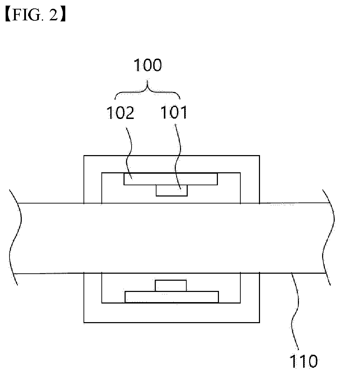

FIG. 2 is a schematic view of the sterilization module according to a first embodiment of the present disclosure.

Referring to Figure 2, the sterilization module 100 may be disposed on the arterial blood line 110.

For example, the sterilization module 100 may be disposed on a catheter that is partially inserted into an arterial blood vessel of a patient. Here, the catheter may be formed of a material including polyurethane, which is permeable to long-wavelength light while being impermeable to short-wavelength UV light.

Accordingly, even when the sterilization module 100 is disposed outside the arterial blood line 110, germicidal light, which is long-wavelength light, can be delivered to the blood in the arterial blood line 110 through the catheter.

As such, the sterilization module 100 may sterilize the blood moving along the arterial blood line 110.

For example, the sterilization module 100 may include a light source 101 and a substrate 102.

The substrate 102 may supply power to the light source 101 to operate the light source 101.

The light source 101 may be a light emitting diode that receives power from the substrate 102 and emits germicidal light.

The germicidal light emitted from the light source 101 may be light in a wavelength band that can kill pathogens while maintaining viability of normal cells at or above a certain level. For example, the germicidal light emitted from the light source 101 may be long-wavelength UV light or visible light that has a peak at a wavelength of about 405 nm.

Here, the normal cells may include white blood cells, red blood cells, and lymphocytes in the blood. In addition, the normal cells may include normal living cells that form other body parts apart from the blood.

The pathogens may include microorganisms such as bacteria, viruses, germs, fungi, protists, and molds. In addition, the pathogens may include highly pathogenic viruses with pandemic potential, such as SARS, influenza, and COVID-19 viruses.

Referring to FIG. 2, the light source 101 and the substrate 102 of the sterilization module 100 may be disposed outside the arterial blood line 110.

For example, the sterilization module 100 may be provided to the sterilization system 10 as a separate device from the arterial blood line 110. That is, the sterilization module 100 may be detachably mounted on the arterial blood vessel line 110. Thus, the sterilization module 100 may also be mounted on other components apart from the arterial blood vessel line 110. That is, the sterilization module 100 can be readily mounted on any component that allows the blood to be sterilized by the sterilization module 100 mounted thereon.

Alternatively, the sterilization module 100 may be secured inside the arterial blood line 110 to be provided integrally with the arterial blood line 100.

In FIG. 2, the sterilization module 100 is shown as including two light source 101-substrate 102 pairs. However, it will be understood that the present disclosure is not limited thereto and the numbers of light sources and substrates may be varied as necessary.

Although not shown in FIG. 1, sensors may be disposed at various locations to detect a condition of the blood, such as blood pressure, changes in blood pressure, and the like.

The sterilization module 100 according to the embodiment emits light in a wavelength band that is determined to be suitable for providing germicidal action through various experiments.

Experiments were conducted to determine which wavelength band could kill only pathogens in blood without damaging normal cells in the blood.

For the experiments, blood was collected from a subject, followed by isolation of living cells from the blood. Thereafter, the living cells isolated from the blood were subjected to light treatment under each condition. Here, the living cells may include normal cells and pathogens.

For an experiment to measure pathogen viability, pathogens were smeared on a bacterial culture medium and cultured at about 35°C to 37°C for one day. Then, colonies of the pathogens were collected from the bacterial culture medium and then were suspended in physiological saline, followed by centrifugation. After centrifugation, the supernatant was discarded and the remaining precipitate was diluted again with physiological saline to prepare a pathogen solution having an appropriate concentration for the experiment. Here, the pathogen was *Staphylococcus aureus.*

For an experiment to measure viability of normal cells, blood was collected from a subject, followed by isolation of lymphocytes from the collected blood. Here, isolation of the lymphocytes was conducted by equilibrium density gradient centrifugation using a Ficoll solution. The isolated lymphocytes were diluted with physiological saline to prepare a lymphocyte solution to be used in the experiment.

The lymphocyte solution and the pathogen solution thus prepared were treated with light in different wavelength bands.

The treated pathogen solution was diluted with physiological saline and then was evenly applied to a bacterial culture medium, followed by culturing. Thereafter, pathogen viability was determined by measuring the number of colonies formed in the culture medium.

In addition, lymphocytes in the treated lymphocyte solution were stained with trypan blue. Thereafter, viability of the normal cells was determined by measuring the number of stained lymphocytes.

### Experiment 1

Experiment 1 is an experiment to determine which light treatment condition allows killing of pathogens without sacrificing viability of normal cells.

In Experiment 1, a first type of light and a second type of light were used.

The first type of light was light having a peak at a wavelength of about 405 nm. In addition, the second type of light was UVA light having a peak in a wavelength band of about 320 to about 400 nm. For example, the second light may be light having a peak at a wavelength of about 365 nm.

Short-wavelength UV light such as UVC and UVB kills pathogens by cutting a chain structure of DNA in cells of the pathogens. That is, short-wavelength UV light is not useful for sterilization of blood due to possibility of mutating DNA in normal cells in the blood, which needs to be injected back into the human body.

Accordingly, short-wavelength UV light, which can also destroy DNA in the normal cells, was excluded from the experiment.

In Experiment 1, energy density-dependent viabilities of normal cells and pathogens were measured by treating the lymphocyte solution and the pathogen solution with the first type of light or the second type of light.

In general, when the viability of living cells drops below 70 in a certain condition, the condition is considered a cytotoxic condition causing damage to the living cells and eventually apoptosis. That is, when the viability of living cells is maintained at 70% or more in a certain condition, the condition can be considered a condition capable of maintaining an environment allowing survival of the living cells without causing toxicity to the living cells.

Accordingly, in the present disclosure, a light treatment condition ensuring a viability of 70% or more for normal cells is considered a condition capable of maintaining an environment allowing survival of the normal cells without causing toxicity to the normal cells.

In addition, in the present disclosure, a light treatment condition ensuring a viability of less than 70% for pathogens can be considered a condition capable of maintaining an environment suitable for killing the pathogens by causing cytotoxicity to the pathogens.

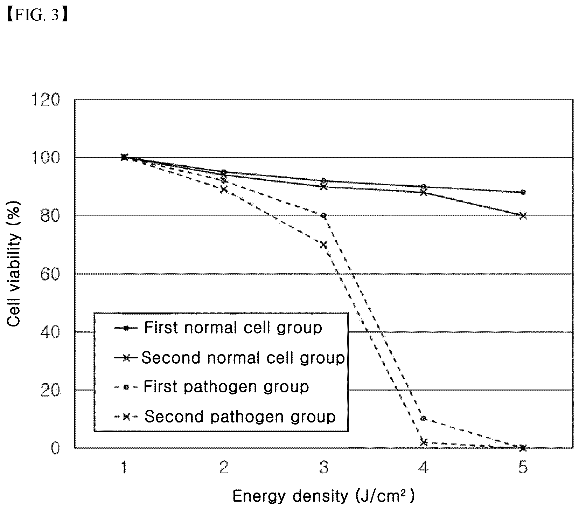

FIG. 3 is a graph showing results of Experiment 1 to determine the viability of normal cells and pathogens depending on light treatment conditions.

In FIG. 3, a first normal cell group is a normal cell group treated with the first type of light and a second normal cell group is a normal cell group treated with the second type of light. In addition, a first pathogen group is a pathogen group treated with the first type of light and a second pathogen group is a pathogen group treated with the second type of light.

Referring to FIG. 3, the first normal cell group, the second normal cell group, the first pathogen group, and the second pathogen group were all gradually decreased in viability with increasing energy density.

However, the first normal cell group and the second normal cell group maintained a viability of 80% or more despite being decreased in viability with increasing energy density.

The first pathogen group and the second pathogen group were sharply decreased in viability at an energy density of 5 J/cm² or more.

The first pathogen group had a viability of about 70% at an energy density of about 6 J/cm² and the second pathogen group had a viability of about 70% at an energy density of 5 J/cm². That is, treatment with the first type of light at an energy density of greater than about 6 J/cm² and treatment with the second type of light at an energy density of greater than about 6 J/cm² correspond to a light treatment condition ensuring a viability of less than 70% for the pathogens.

In particular, the viability of the first pathogen group and the second pathogen group was decreased to less than 20% at an energy density of 10 J/cm². In addition, the viability of the first pathogen group and the second pathogen group approached 0% at an energy density of 20 J/cm².

Experiment 1 shows that treatment with the first type of light, that is, light having a peak at a wavelength of about 405 nm, at an energy density of greater than about 6 J/cm² and treatment with the second type of light, that is, UVA, at an energy density of greater than about 5 J/cm² can satisfy both the desired level of viability of normal cells and the desired level of pathogen killing capacity.

In particular, it can be seen that both the first type light and the second type light have high killing capacity against the pathogens while ensuring high viability for the normal cells at an energy intensity of about 10 J/cm².

### Experiment 2

Experiment 2 is an experiment to determine a degree of DNA damage to living cells depending on the type of light.

In order to determine the degree of DNA damage, living cells were isolated from blood of a healthy subject and blood of a diseased subject. The isolated living cells were subjected to light treatment under each condition. The living cells used in Experiment 2 were lymphocytes.

In this experiment, a comet assay was conducted to measure DNA strand breaks in living cell groups subjected to light treatment under respective conditions. Specifically, the percentage of DNA in a comet tail resulting from DNA strand breaks ("tail intensity") was measured in the comet assay. Then, the median of the tail intensity measurements was analyzed to determine the degree of DNA damage to the living cells.

In Experiment 2, a control group was a living cell group not subjected to light treatment. A first experimental group was a living cell group subjected to treatment with the first type of light, that is, light having a peak at a wavelength of about 405 nm, at an energy density of 5 J/cm². A second experimental group was a living cell group subjected to treatment with the first type of light at an energy density of 10 J/cm². A third experimental group was a living cell group subjected to treatment with the second type of light, that is, UVA, at an energy density of 5 J/cm². A fourth experimental group was a living cell group subjected to treatment with the second type of light at an energy density of 10 J/cm².

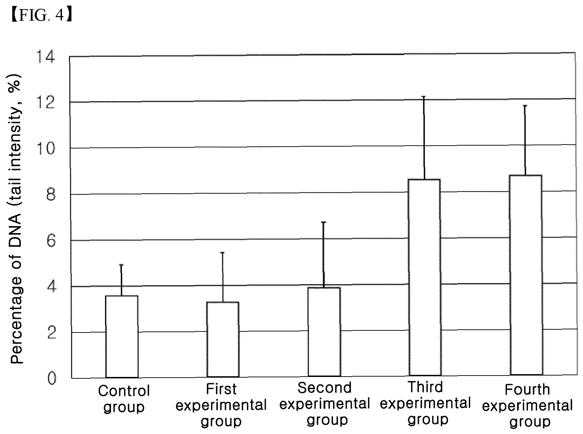

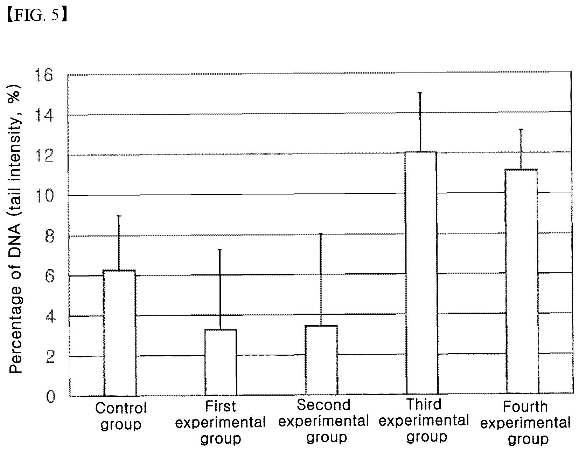

In the graphs of FIG. 4 and FIG. 5, the x-axis represents light treatment conditions and the y-axis represents the percentage of DNA in a comet tail resulting from DNA strand breaks ("tail intensity") in the living cells.

FIG. 4 is a graph showing the degree of DNA damage to living cells in blood of a healthy subject depending on light treatment conditions.

Referring to FIG. 4, the control group, the first experimental group, and the second experimental group had tail intensities of less than about 8%, which were similar to one another. That is, regardless of energy density levels, the living cell groups treated with the first type of light exhibited similar levels of tail intensity to the living cell group not subjected to light treatment.

However, the third experimental group and the fourth experimental group had significantly higher tail intensities than the control group. Specifically, the control group had a tail intensity of less than 6%, whereas the third experimental group and the fourth experimental group had tail intensities of greater than 8%.

The results of FIG. 4 show that light-induced DNA damage occurred in the living cell groups treated with the second type of light, regardless of energy density levels.

That is, when the blood of the healthy subject was treated with the first type of light, the degree of DNA damage to the living cells was similar to that in the living cells not subjected to light treatment. Conversely, when the blood of the healthy subject was treated with the second type of light, the degree of DNA damage to the living cells was significantly higher than that in the living cells not subjected to light treatment.

FIG. 5 is a graph showing the degree of DNA damage to living cells of blood of a diseased subject depending on light treatment conditions.

Here, the disease of the subject may include any disease, such as hereditary diseases, diseases caused by infection, and diseases caused by *in vivo* inflammatory response.

Referring to FIG. 5, the control group, the first experimental group, and the second experimental group had tail intensities of 8% or less.

The tail intensities of the first experimental group and the second experimental group were lower than that of the control group. However, considering error ranges, there was no significant difference in tail intensity between the control group and the first and second experimental groups.

The third experimental group and the fourth experimental group had tail intensities of greater than 8%, specifically greater than 10%.

That is, the third experimental group and the fourth experimental group had significantly higher tail intensities than the control group.

When the blood of the diseased subject was treated with the first type of light, the degree of DNA damage to the living cells in the blood was similar to that in the living cells not subjected to light treatment. Conversely, when the blood of the healthy subject was treated with the second type of light, the degree of DNA damage to the living cells in the blood was significantly higher than that in the living cells not subjected to light treatment.

Experiment 2 shows that both blood from a healthy subject and blood from a diseased subject are hardly damaged by the first type of light and are significantly damaged by the second type of light.

Therefore, it can be seen that the second type of light, that is UVA, is not suitable for sterilizing blood due to the disadvantage of causing DNA damage to living cells despite having high killing capacity against pathogens without sacrificing viability of the living cells.

### Experiment 3

Experiment 3 is an experiment to determine the degree of DNA damage to living cells dependent on the energy density of the first type of light.

Like Experiment 2, Experiment 3 used living cell groups extracted from blood of a healthy subject and living cell groups extracted from blood of a diseased subject. Living cells used in Experiment 3 were lymphocytes.

In Experiment 3, a control group was a living cell group not subjected to light treatment. A first experimental group was a living cell group treated with the first type of light, that is, light having a peak at a wavelength of about 405 nm, at an energy density of 5 J/cm². A second experimental group was a living cell group treated with the first type of light at an energy density of 10 J/cm². A third experimental group was a living cell group treated with the first type of light at an energy density of 20 J/cm².

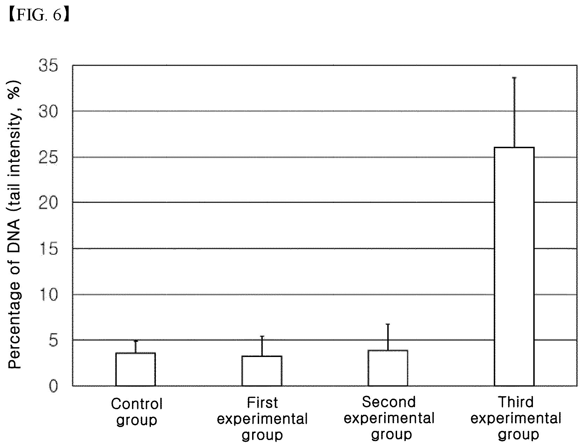

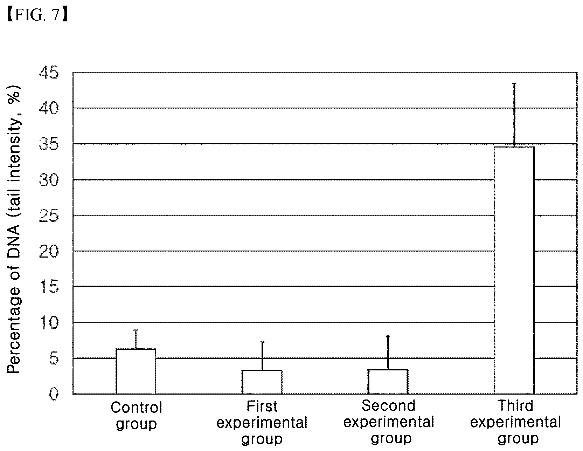

In the graphs of FIG. 6 and FIG. 7, the x-axis represents the light treatment conditions and the y-axis represents the percentage of DNA in a comet tail resulting from DNA strand breaks ("tail intensity") in the living cells.

FIG. 6 is a graph showing the degree of DNA damage to the living cells in the blood of the healthy subject depending on the energy density of the first type of light.

Referring to FIG. 6, the control group, the first experimental group, and the second experimental group had tail intensities of 8% or less.

In addition, the control group, the first experimental group, and the second experimental group had similar levels of tail intensity.

The third experimental group had a tail intensity of greater than 8%, specifically greater than 25%.

In addition, the tail intensity of the third experimental group was significantly higher than that of the control group.

That is, it can be seen that treating blood of a healthy subject with the first type of light at an energy density of 20 J/cm² or more can cause significant damage to living cells in the blood.

FIG. 7 is a graph showing the degree of DNA damage to living cells in blood of a diseased subject depending on the energy density of the first type of light.

Referring to FIG. 7, the control group, the first experimental group, and the second experimental group had tail intensities of 8% or less.

In addition, the control group, the first experimental group, and the second experimental group had similar levels of tail intensity.

The third experimental group had a tail intensity of greater than 8%, specifically greater than 30%.

In addition, the tail intensity of the third experimental group was significantly higher than that of the control group.

That is, it can be seen that treating blood of a diseased subject with the first type of light at an energy density of 20 J/cm² or more can cause significant DNA damage to living cells in the blood.

As such, Experiment 3 shows that treatment with the first type of light at an energy density of 20 J/cm² or more causes significant DNA damage to living cells in the blood of both healthy and diseased subjects.

As describe above, the living cells used in Experiments 2 to 3 were lymphocytes, which are normal cells in the blood. That is, Experiments 2 to 3 show that treatment with the first type of light at an energy density of less than 20 J/cm² causes little or no DNA damage to the normal cells in the blood. Conversely, treatment with the first type of light at an energy density of 20 J/cm² or more can cause significant DNA damage to the normal cells in the blood.

In conclusion, Experiments 1 to 3 show that treatment with light having a peak at a wavelength of about 405 nm at an energy density of greater than about 6 J/cm² to less than 20 J/cm² can ensure both a viability of 70% or more for normal cells and killing of pathogens without causing DNA damage to the normal cells.

In one embodiment, the sterilization module 100 of FIG. 1 and FIG. 2 may emit germicidal light having a peak at a wavelength of about 405 nm at an energy density of greater than about 6 J/cm² to less than 20 J/cm². That is, the sterilization module 100 according to the embodiment can effectively kill pathogens while minimizing damage to normal cells in blood through delivery of the germicidal light to the blood.

Further, treatment with the germicidal light from the sterilization module 100 at an energy density of about 10 J/cm² to less than 20 J/cm² can ensure significantly improved killing action against pathogens while minimizing damage to normal cells in blood.

It is thought that treatment with the germicidal light under such conditions will act equally on pathogens and normal cells in other body tissue apart from blood.

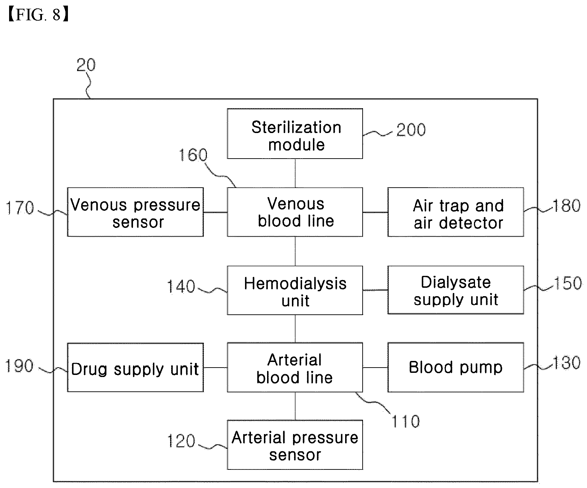

FIG. 8 is a block diagram of a sterilization system according to a second embodiment of the present disclosure.

Referring to FIG. 8, the sterilization system 20 according to the second embodiment may include the same components as the sterilization system (10 in FIG. 1) according to the first embodiment.

However, the sterilization system 20 according to the second embodiment differs from the sterilization system (10 in FIG. 1) according to the first embodiment in that a sterilization module 200 of the sterilization system 20 is disposed at a different location than the sterilization module 100 of the sterilization system 10.

Thus, for details of other components and features apart from the location of the sterilization module 200, see description as to the sterilization system (10 in FIG. 1) according to the first embodiment.

Referring to FIG. 8, the sterilization module 200 may be disposed on the venous blood line 160.

In this embodiment, the sterilization module 200 may deliver germicidal light to blood with waste matter removed therefrom through dialysis to kill pathogens in the blood. That is, the sterilization module 200 may kill pathogens in blood subjected to dialysis before injecting the blood back into the vein of a patient.

In the first embodiment of FIG. 1, the sterilization module 100 is disposed on the arterial blood line 110, whereas, in the second embodiment of FIG. 8, the sterilization module 100 is disposed on the venous blood line 160. However, the location of the sterilization module 100 or 200 is not limited thereto. The sterilization module 100 or 200 may be disposed at any location of the sterilization system 10 or 20, at which the sterilization module 100 or 200 can deliver the germicidal light to the blood.

Although the sterilization systems 10, 20 are illustrated in FIG. 1 and FIG. 8, at least some components of the sterilization system 10 or 20 may be included in a hemodialysis device.

For example, the arterial blood line 110, the arterial pressure sensor 120, the blood pump 130, the hemodialysis unit 140, the venous pressure sensor 170, the venous blood line 160, and the air trip and the air detector 180 may be included in the hemodialysis device. Here, the drug supply unit 190, the dialysate supply unit 150, and the sterilization module 100 or 200 may be disposed outside the hemodialysis device.

Alternatively, all the components of the sterilization system 10 or 20 may be included in the hemodialysis device.

In addition, the hemodialysis device is not necessarily composed of only the components of the sterilization system 10 or 20 described in FIG. 1 or FIG. 8. That is, components constituting the hemodialysis device may be varied as necessary.

As described above, the sterilization system according to the embodiments includes the sterilization module emitting the germicidal light. In addition, the germicidal light emitted from the sterilization module can kill pathogens in blood, excluding normal cells in the blood.

Accordingly, the sterilization module and the sterilization system according to the embodiments can provide healthy blood to a patient by killing the pathogens while minimizing damage to the normal cells.

The present disclosure has been described by way of an example in which the sterilization module is applied to a hemodialysis device to form the sterilization system.

However, it will be understood that the present disclosure is not limited to applying the sterilization module to the hemodialysis device.

For example, the sterilization module may be applied to a surgical system. Here, the sterilization module may be mounted in an operating room or on surgical equipment. The sterilization module can kill pathogens at or around a surgical site without causing damage to normal living cells through delivery of the germicidal light to the surgical site. Accordingly, when the sterilization module according to the embodiments is applied to the surgical system, it is possible to reduce infection by pathogens during surgery.

In addition, the sterilization module may treat or help to manage infections through delivery of the germicidal light to body tissue infected with pathogens.

Although some embodiments have been described herein with reference to the accompanying drawings, it should be understood that these embodiments are provided for illustration only and are not to be construed in any way as limiting the present disclosure. Therefore, the scope of the present disclosure is not limited to the above embodiments and should be defined only by the accompanying claims and equivalents thereto.

## Claims

1. A sterilization module comprising:
a light source emitting germicidal light to kill pathogens in living cells, the living cells comprising normal cells and the pathogens,
wherein the germicidal light is light in a wavelength band capable of ensuring a viability of 70% or more for the normal cells and a viability of less than 70% for the pathogens.

2. The sterilization module according to claim 1, wherein the germicidal light is light in a wavelength band capable of ensuring a viability of 80% or more for the normal cells and a viability of 20% or less for the pathogens.

3. The sterilization module according to claim 1, wherein the germicidal light ensures a tail intensity of less than 8% for the living cells, the tail intensity being a percentage of DNA in a comet tail resulting from DNA strand breaks in the living cells.

4. The sterilization module according to claim 1, wherein the germicidal light is light having a peak at a wavelength of 405 nm.

5. The sterilization module according to claim 4, wherein the light source delivers the germicidal light to the living cells at an energy density of greater than 6 J/cm² to less than 20 J/cm².

6. The sterilization module according to claim 5, wherein the light source delivers the germicidal light to the living cells at an energy density of 10 J/cm².

7. The sterilization module according to claim 1, wherein the light source delivers the germicidal light to body tissue or blood discharged from a human body.

8. A sterilization system comprising:
a sterilization module emitting germicidal light to kill pathogens in living cells, the living cells comprising normal cells and the pathogens,
wherein the germicidal light is light in a wavelength band capable of ensuring a viability of 70% or more for the normal cells and a viability of less than 70% for the pathogens.

9. The sterilization system according to claim 8, wherein the germicidal light is light in a wavelength band capable of ensuring a viability of 80% or more for the normal cells and a viability of 20% or less for the pathogens.

10. The sterilization system according to claim 8, wherein the germicidal light ensures a tail intensity of less than 8% for the living cells, the tail intensity being a percentage of DNA in a comet tail resulting from DNA strand breaks in the living cells.

11. The sterilization system according to claim 8, wherein the germicidal light is light having a peak at a wavelength of 405 nm.

12. The sterilization system according to claim 11, wherein the light source module delivers the germicidal light to the living cells at an energy density of greater than 6 J/cm² to less than 20 J/cm².

13. The sterilization system according to claim 12, wherein the light source module delivers the germicidal light to the living cells at an energy density of 10 J/cm².

14. The sterilization system according to claim 8, wherein the sterilization module delivers the germicidal light to body tissue or blood discharged from a human body.

15. The sterilization system according to claim 14, further comprising:
a hemodialysis device for dialysis of the blood discharged from the human body.
